# EUROPEAN PATENT SPECIFICATION

(11) **EP 4 517 100 B1**
(45) Date of publication and mention of the grant of the patent: **15.10.2025**
(21) Application number: 24197305.6
(22) Date of filing: 29.08.2024
(51) Int. Cl.: F04D 29/02, C02F 1/467, F04D 29/42, A61L 2/03

(54) **PREVENTION OF MICROBIOLOGICAL GROWTH IN PROCESSING EQUIPMENT HAVING A ROTATING HOUSING**
VERHINDERUNG VON MIKROBIOLOGISCHEM WACHSTUM IN VERARBEITUNGSAUSRÜSTUNG MIT EINEM ROTIERENDEN GEHÄUSE
PRÉVENTION DE LA CROISSANCE MICROBIOLOGIQUE DANS UN ÉQUIPEMENT DE TRAITEMENT AYANT UN BOÎTIER ROTATIF

(30) Priority: 29.08.2023 EP 23193833
(43) Date of publication of application: 05.03.2025
(73) Proprietor: EPFF Electrical Pipe For Fluid transport AB, 736 32 Kungsör (SE)
(72) Inventor: ANDERSSON, Thomas, 736 32 KUNGSÖR (SE)
(74) Representative: Brann AB

(56) References cited:
- WO-A1-2021/069395
- FR-A1- 2 697 443
- GB-A- 201 907
- US-A1- 2019 249 815

## Description

### Technical field

The technology proposed herein relates to the prevention of microbiological growth in food processing equipment.

### Background

Microbiological growth in food processing equipment is a recognized problem, for example in diary processing. The feed that is processed have a high nutrient content and the rate of microbiological growth in the equipment is typically high. Extensive biofilms can form in a few hours. Frequent cleaning is typically required to prevent a drop in quality of the produced product or in the performance of the equipment. For example, microbiological growth may contaminate the produced product, or it may obstruct the flow of the fluid within the equipment. Typically, production must be interrupted for cleaning and chemicals are commonly used, which contributes to a decreased efficiency and increased production costs.

Microbiological growth is particularly a problem in equipment that that cannot easily be disassembled for manual cleaning. Equipment having rotating components, such centrifuge separators are typically difficult to disassemble.

US2019/249815A1 discloses a system for preventing microbiological growth in a piping system.

### Object of the proposed technology

The proposed technology aims at preventing or reducing microbiological growth, such as the formation of biofilms, in food processing equipment. A particular object of the proposed technology is to reduce microbiological growth in equipment having rotating housing.

### Summary

In a first aspect of the proposed technology, a system is proposed that comprises: a housing, or container, and an electric power source, or electric power supply. The housing is electrically conductive and arranged to, or configured to, hold, or contain, a fluid, the power source is arranged to supply an electric current, and/or an electric potential, and the housing and the electric power source are operationally coupled in an electric circuit arranged to lead the current from the electric power source through, or via, the housing, and/or to establish the potential over the housing. The electric current, and/or the electric potential, is configured to inhibit, reduce, or prevent, microbiological growth within the housing, or on the housing.

The system further comprises: a static element, or a stationary element. The static element may be located at least partly within the housing. The housing is a rotating housing, or rotary housing. It is understood that the housing may be arranged to rotate around a housing axis, or first rotational axis.

It is understood that the intention of the system is to prevent microbiological growth in the housing. It is further understood that the housing may form part of a food processing equipment.

It is further understood that the housing has a portion that is exposed to the fluid, and the electric circuit may be arranged to lead the current from the electric power source through, or via, the portion exposed to the fluid. It is further understood that the current is adapted to inhibit microbiological growth on the housing or on the portion of the housing exposed to the fluid.

It is specified that the electric circuit is arranged to lead the current through the housing. It is understood that the housing extends in all dimensions and that the current may be evenly or unevenly distributed in the housing. It is further understood that at least a portion of the housing forms part of the electric circuit. This means that the current passes through, or via, this portion. The complete housing may form part of the circuit, which means that the current passes through the complete housing.

Throughout these specifications, if a first element is electrically coupled to a second element there may be further conductive elements between them, and if a first element is electrically connected to a second element there are no conductive elements between them.

The fluid may be susceptible to microbiological growth. The fluid may contain water. The fluid may be a liquid, such as raw milk or raw juice. It is understood that the fluid may contain solid particles. The fluid may be a paste, or a minced or pureed product.

The electric circuit may be connected to ground. Worded differently, the housing may be electrically coupled to the power source via ground, or the electric circuit may incorporate ground. Ground may be a signal ground or earth ground. For example, ground may incorporate an electrically conductive structure connected to and supporting the housing.

The power source may have a first terminal and a second terminal. The first terminal may supply the current or the potential and the second terminal may be, coupled, or connected, to ground. In this context, it is understood that the potential is relative to ground. The power source may be a single-phase power source. This means that it has a single terminal supplying the current or the potential, for example by the first terminal.

The current may be an alternating current. An alternating current is understood to periodically change direction. The current may have a square waveform. The current may be below 10 mA, below 1 mA, or below 0.1 mA. The current may be greater than 0.01 mA. The current may be in the range 10 mA to 1 mA, 1 mA to 0.1 mA, or 0.1 mA to 0.01 mA. Here, the current is understood as the absolute maximum over a period. It has been found that this current is sufficient for inhibiting microbiological growth. The alternating current may have a frequency below 100 Hz, below 10 Hz, or below 1 Hz. The frequency may be greater than 0.1 Hz. The alternating current may be supplied at a peak voltage below 120 V, in the range 10 V to 100 V, or in the range 20 V to 90 V, or in the range 30 V to 70 V.

Alternatively to the current being an alternating current, the current may be a direct current. A direct current is understood to have a constant direction. The direct current may be a pulsed current. It is understood that no current is supplied between pulses. The pulsed current may have a regular pulse frequency. The pulse frequency may be below 100 Hz, below 10 Hz, or below 1 Hz. The pulse frequency may be greater than 0.1 Hz. The direct current may have a duty cycle in the range 60% to 40%, or about 50%. For example, the current may be supplied with a pulse length of 500 ms with a pulse separation of 500 ms. The pulsed current may be supplied at a peak voltage below 120 V, in the range 10 V to 100 V, or in the range 20 V to 90 V, or in the range 30 V to 70 V.

The housing may be of metal, such as steel. The housing may form, or enclose, an inner space for holding, or retaining, the fluid. The housing may have a wall forming the inner space for holding the fluid. It is understood that the wall is exposed to the fluid. The wall may be electrically conductive. For example, the wall may be of metal. It is understood that the housing, or wall, may be composed of several components that are connected, or joined, together. The wall may be, or form part of the abovementioned portion of the housing.

The housing may have, or form, an inlet, or feed nozzle, for receiving, or introducing, the fluid into the housing. The housing may have, or form, an outlet, or discharge nozzle, for discharging, or dispelling, the fluid from the housing. The inlet and the outlet may be located at opposite sides, or ends, of the housing. It is understood that the housing may be exposed to the fluid between the inlet and the outlet. The electric circuit may be connected to the housing at the inlet and at the outlet. This contributes to inhibit microbiological growth between the inlet and the outlet. If the inlet and the outlet are located at opposite sides of the housing, microbiological growth in the complete housing may be inhibited.

The system may further comprise a support structure that rotationally supports the housing, for example by way of one or more support bearings. The housing may be electrically connected, or coupled, to the support structure, for example via the support bearings. Alternatively, it may be electrically insulated from the support structure.

The housing may comprise a housing shaft. The housing shaft may be centred on the housing axis. The housing shaft may be a driven shaft, or transmission shaft. For example, the system may comprise a housing motor, wherein the housing motor is coupled to and arranged to rotate the housing shaft. It is understood that there may be parts of the housing, for example the housing shaft, that does not hold the fluid.

It is specified that the housing is electrically conductive. This means that the housing shaft is electrically conductive. For example, the housing shaft may be of metal, such as steel. It is specified that the electric circuit is arranged to lead the current through the housing. The electric circuit may be further arranged to lead the current through, or via, the housing shaft. Worded differently, the housing shaft may form part of the abovementioned electric circuit. Additionally, it may form part of the electric network described below.

Any of the abovementioned inlet or outlet may be formed at, or by, the housing shaft. The housing shaft may form a fluid conduit that connects the inlet or outlet to the inner space. Worded differently, the fluid conduit may be arranged to, or configured to, lead the fluid to the inner space. This way, the inlet or outlet can be spaced apart from the inner space.

The system may further comprise a feed pipe connected to the inlet of the housing and arranged to, or configured to, lead the fluid to the inlet. Similarly, the system may further comprise a discharge pipe connected to the outlet of the housing and arranged to, or configured to, lead the fluid from the outlet.

The feed pipe and/or the discharge pipe may be electrically conductive. For example, they may be of metal, such as steel. The feed pipe and/or the discharge pipe may be electrically connected to the housing, for example by directly contacting the housing. The feed pipe and/or the discharge pipe may form part of the abovementioned electric circuit. The electric circuit may be arranged to lead the current through, or via, the feed pipe and/or the discharge pipe, and/or to establish the potential over the housing via the feed pipe and/or the discharge pipe. The electric circuit may be arranged to lead the current through and/or to establish the potential over at least a portion of the feed pipe and/or over at least a portion of the discharge pipe. This means that there may be parts of the feed pipe and/or discharge pipe through which the current does not run. The electric circuit may be arranged to lead the current through and/or to establish the potential over the complete feed pipe and/or the discharge pipe.

It is specified that the static element may be located at least partly within the housing. More specifically, the static element may be located at least partly within the inner space. Alternatively, the complete static element may be located outside the housing, or more specifically outside the inner space.

It is specified above that the housing is a rotating housing arranged to rotate around a housing axis. The housing axis may extend through the static element. Worded differently, the static element may be centred on the housing axis, or the static element and the housing shaft may be concentric. With this configuration, it is understood that the housing is arranged to rotate relative to the static element.

The static element may extend from within the housing, or the inner space, to outside the housing. The static element may extend through, or via, the housing shaft. Worded differently, the static element may in part be located within the abovementioned inner space formed by the housing. It may further be accessible from outside the housing. It is understood that the static element may be a rigid self-supporting structure.

The static element may form an inlet for introducing the fluid into the housing, or in the inner space formed by the housing. The inlet may be located within the housing or in the inner space. The static element may form an outlet for dispelling the fluid from the housing, or from inner space formed by the housing. It is understood that the inner space is in fluid communication with the inlet and the outlet. The outlet may be located within the housing or in the inner space. Alternatively, the housing may extend into the static element, and the outlet may be located outside the housing or outside the inner space.

It is specified above that the housing is a rotating housing. The stationary element may be a supporting shaft, wherein the supporting shaft extends from within the housing, or the inner space, to outside the housing and rotationally supports the housing. The supporting shaft may be centred on the housing axis.

The static element may be electrically conductive. For example, the static element may be of metal, such as steel. The static element may form part of the abovementioned electric circuit. This way, the electric current, and/or the electric potential, is further configured to inhibit, reduce, or prevent microbiological growth on the static element. The electric circuit may be arranged to lead the current through, or via, both the housing and the static element, and/or to establish the potential over the housing and the static element. The electric circuit may be arranged to lead the current through and/or to establish the potential over at least a portion of the housing and/or over at least a portion of the static element. This means that there may be parts of the housing and/or the static element through which the current does not run. The electric circuit may be arranged to lead the current through and/or to establish the potential over the complete housing and/or the complete static element.

The static element and the housing may be electrically coupled in an electric network. It is understood that the electric network forms part of the abovementioned electric circuit. The electric network may be arranged to distribute the current between the housing and the static element. For example, the housing and the static element may be arranged in parallel in the electric network. This means that in the electric network, a first portion of the current is conducted through, or via, the housing and a second portion of the current is conducted through, or via, the static element.

The static element may be electrically coupled to the housing. The static element may contact the housing in operation. The system may comprise a first internal connector, or first internal connector arrangement, that electrically couples the static element to the housing. The system may further comprise a second internal connector, or second internal connector arrangement, that electrically couples the static element to the housing.

It is specified above that the housing is a rotating housing, and any of the first internal connector and the second internal connector may be a rotary electrical connector, or electrical rotary joint. The first internal connector and the second internal connector may be spaced apart along the housing axis. They may be centred on the housing axis.

For example, any of the internal connectors may comprise a slip ring connecting the static element and the housing. Additionally or alternatively, any of the internal connectors may comprise a bearing, such as a metal rolling-element bearing or a metal plain bearing connecting the static element and the housing. Additionally or alternatively, any of the internal connectors may comprise a metal brush or carbon brush connecting the static element and the housing. Additionally or alternatively, any of the internal connectors may comprise a wear ring attached to the static element or the housing and connecting the static element and the housing. Additionally or alternatively, any of the internal connectors may comprise an electrically conductive seal, for example of metal, connecting the static element and the housing. It is understood that the seal may be arranged to a inhibit, or prevent, a fluid flow between the static element and the housing.

The static element may be connected to the electric circuit via the housing. For example, the abovementioned second portion of the current may pass through, or via, the housing before and/or after passing through, or via, the static element.

The housing may be connected to the electric circuit via the static element. For example, the abovementioned first portion of the current may pass through, or via, the static element before and/or after passing through, or via, the housing.

The system may further comprise: a first connector, or first connector arrangement, wherein the housing is electrically coupled to the power source via the first connector. The first connector may be connected to the housing or the static element. This means that the first connector forms part of the abovementioned electric circuit. If the first connector is connected to the housing, the first connector may be a rotary electrical connector. If the first connector is connected to the static element, the first connector may be a static connector. The system may further comprise: a second connector, or second connector arrangement, wherein the housing is electrically coupled to the power source via the second connector. The second connector may be connected to the housing or the static element. This means that the second connector forms part of the abovementioned electric circuit. If the second connector is connected to the housing, the second connector may be a rotary electrical connector. If the second connector is connected to the static element, the second connector may be a static connector. The abovementioned support structure may constitute, or form, the second connector, and the support structure may be connected to ground.

The first connector and the second connector may be positioned on opposite sides of the housing, or the inner space. The abovementioned inlet and/or outlet may be located between the first connector and the second connector. This contributes to inhibit microbiological growth withing a greater part of the housing.

The abovementioned first internal connector and/or second internal connector may be located between the first connector and the second connector. This contributes to a inhibit microbiological growth over a greater part of the static element.

The system may further comprise an electric first wire, and the first connector may be connected to the power source by the first wire. The first wire may be connected to the abovementioned first terminal of the power source. Alternatively, the first connector and the power source may be connected via ground, for example via the abovementioned support structure. The system may further comprise an electric second wire, and the second connector may be connected to the power source by the second wire. The second wire may be connected to the abovementioned second terminal of the power source. Alternatively, the second connector and the power source may be connected via ground, for example via the abovementioned support structure. It is understood that the first connector and the second connector may not both be connected to the power source via ground.

It is specified above that the housing is a rotating housing. The first connector may be connected to the housing, or more specifically to the housing shaft. Similarly, the second connector may be connected to the housing, or more specifically to the housing shaft. With these configurations, any of the first connector and the second connector may be a rotary electrical connector, or an electrical rotary joint.

It is further specified above that the system may comprise a static element. If both the first connector and the second connector are connected to the housing, the current may then be distributed to the static element via the abovementioned first internal connector and second internal connector. The first connector and the second connector may be spaced apart along the housing axis. This contributes to a inhibit microbiological growth over a greater part of the static element, particularly if the first internal connector and the second internal connector are spaced apart along the housing axis.

For example, the rotary electrical connector may comprise a slip ring connecting to the housing, or to the housing shaft. Additionally or alternatively, it may comprise a bearing, such as a metal rolling-element bearing or a metal plain bearing connecting to the housing, or to the housing shaft. For example, the bearing may have an inner race connected to the housing, or to the housing shaft and an outer race connected to the abovementioned support structure. Additionally or alternatively, the rotary electrical connector may comprise a metal brush or carbon brush connecting to the housing, or to the housing shaft. Additionally or alternatively, it may comprise a wear ring connecting to the housing, or to the housing shaft. Additionally or alternatively, the rotary electrical connector may comprise an electrically conductive seal, for example of metal, connecting to the housing, or to the housing shaft.

It is specified above that the housing is a rotating housing and that the system additionally may comprise a static element. The first connector may be connected to the static element. Similarly, the second connector may be connected to the static element. In these configurations, any of the first connector and the second connector may be a static connector, or a fixed connector. This means that the connector cannot move relative to the static element. For example, the static connector may be a ring lug connector secured to the static element by a screw. If both the first connector and the second connector are connected to the static element, the current may then be distributed to the housing via the abovementioned first internal connector and second internal connector.

It is understood the first connector may be connected to the static element and the second connector may be connected to the housing, and vice versa.

The housing, and optionally the static element, may form part of a centrifuge separator. Wording the first aspect of the proposed technology differently, the system comprises: an electric power source, or electric power supply, and a centrifuge separator, or centrifugal rotor, having a housing. The housing is rotating housing. The housing is electrically conductive and arranged to hold, or contain, a fluid. The power source is arranged to supply an electric current, and/or an electric potential, and the housing and the electric power source are operationally coupled in an electric circuit arranged to lead the current from the electric power source through, or via, the housing, and/or to establish the potential over the housing. The electric current, and/or the electric potential, is configured to inhibit, reduce, or prevent microbiological growth within the housing, or on the housing. As specified above, it is understood that the fluid may be a liquid.

It is understood that centrifuge separators encompass centrifuge clarifiers. It is further understood that the system may have any of the features described above. It is specified above that the separator has a housing. This feature is commonly called a bowl or a rotor body. It is further specified above that the housing may have a housing shaft. In the technical field of centrifuge separators, this feature is commonly called a bowl spindle, or simply a spindle. Here, the spindle is understood as a part of the housing. The housing axis around which the housing can rotate may be vertical.

It is further understood that the properties, or composition, of the liquid introduced into the centrifuge separator may differ from those of the liquids discharged from the centrifuge separator. The properties, or composition, may also vary within the housing. For example, the centrifuge separator may form lighter and heavier phases of the liquid that are discharged separately.

The housing may have a rotational symmetry, or be rotationally symmetric, relative to the housing axis. The housing may be an assembled structure, for example it may have a lower bowl body and an upper bowl body. Here, the positions of the lower bowl body and the upper bowl body are relative to a vertical housing axis. The lower bowl body and the upper bowl body may be annular and centred on the housing axis. Additionally, they may have rotational symmetry, or be rotationally symmetric, relative to the housing axis. The abovementioned housing shaft, or spindle, may be attached to, or fixed to, the lower bowl body. It is understood that the lower bowl body and the upper bowl body are electrically coupled or connected. The lower bowl body and the lower bowl body may form, or enclose, the abovementioned inner space. Alternatively, the housing may further have a sliding piston, or sliding bowl bottom, arranged to slide relative to the lower bowl body parallel to the housing axis. It is understood that the sliding piston is electrically coupled or connected to the lower bowl body and/or to the upper bowl body. The sliding piston and the upper bowl body may form, or enclose, the abovementioned inner space. The sliding piston may have an upper position in which it seals to the upper bowl body and a lower position in which the fluid, or sludge accumulated from the fluid, can be ejected from the inner space between the upper bowl body and the sliding piston. The lower bowl body may form a plurality of ejection ports, or sludge ejection ports, through which fluid, or accumulated slutch, can pass after being ejected from the inner space.

It is specified above that the inlet or outlet may be formed by the housing shaft and that the housing shaft may form a fluid conduit that connects the inlet or outlet to the abovementioned inner space.

The centrifuge separator, or more generally the system, may have an inlet, or feed nozzle, for receiving, or introducing, the fluid into the housing or the inner space. The centrifuge separator, or more generally the system, may have an outlet, or discharge nozzle, for discharging, or dispelling, the fluid from the housing or the inner space. The centrifuge separator, or more generally the system, may further have an additional outlet, or discharge nozzle, for discharging, or dispelling, the fluid from the housing or the inner space.

The system, or centrifuge separator, may comprise a static element. The static element may be located at least partly within the housing and form the inlet for introducing the fluid into the housing, as specified above. The inlet may be located within the housing or in the inner space. For example, the static element may form an inlet pipe extending from outside the housing into the housing, the inner space, or the inlet chamber further described below. The inlet pipe may be centred on the housing axis. The inlet pipe may have an outer open end for receiving the fluid and an inner open end constituting the inlet. It is understood that the outer open end is located outside the housing and that the inner open end is located inside the housing. Further specifications of the static element are presented above.

The static element may form an outlet for dispelling the fluid from the housing or from the inner space, as specified above. The housing and the static element may jointly form a pump, such as a centripetal pump, arranged to pump the fluid through the outlet at a rotation of the housing. For example, the housing may form a paring chamber, and the static element may form a paring disc that cooperates with the paring chamber to pump the fluid through the outlet at a rotation of the housing. It is understood that the paring chamber rotates with, or is fixed to, the rest of the housing and that the paring disc is stationary with the rest of static element. For example, the static element may form an outlet pipe. The outlet pipe may be centred on the housing axis. The outlet pipe may be concentric with and surround the inlet pipe. The outlet pipe has an inner open end coupled to the pump, or the paring disc, and an outer open end constituting the outlet. The outlet pipe may be connected to, or electrically couplet to, the inlet pipe.

The static element may form the additional outlet for discharging the fluid from the housing or from the inner space. The housing and the static element may jointly form an additional pump, such as a centripetal pump, arranged to pump the fluid through the outlet at a rotation of the housing. For example, the housing may form an additional paring chamber, and the static element may form an additional paring disc that cooperates with the additional paring chamber to pump the fluid through the additional outlet at a rotation of the housing. It is understood that the additional paring chamber rotates with, or is fixed to, the rest of the housing and that the additional paring disc is stationary with the rest of the static element. For example, the static element may form an additional outlet pipe concentric with and surrounding the outlet pipe. The additional outlet pipe may be centred on the housing axis. The additional outlet pipe has an inner open end coupled to the additional pump, or the additional paring disc, and an outer open end constituting the additional outlet. The additional outlet pipe may be connected to, or electrically couplet to, the outlet pipe.

It is specified above that the static element may form an outlet for dispelling the fluid from the housing or from the inner space. It is further specified above that the housing may extend into the static element, and the outlet may be located outside the housing or outside the inner space. The housing and the static element may jointly form a pump, such as a centrifugal pump, arranged to pump the fluid through the outlet at a rotation of the housing. For example, the housing may form an impeller, and the static element may form a volute casing that cooperates with the impeller to pump the fluid through the outlet at a rotation of the housing. It is understood that the impeller rotates with, or is fixed to, the rest of the housing and that the volute casing is stationary with the rest of static element.

The static element may form an additional outlet for discharging the fluid from the housing or from the inner space. The housing and the static element may jointly form an additional pump, such as a centrifugal pump, arranged to pump the fluid through the outlet at a rotation of the housing. For example, the housing may form an additional impeller, and the static element may form an additional volute casing that cooperates with the additional impeller to pump the fluid through the additional outlet at a rotation of the housing. It is understood that the additional impeller rotates with, or is fixed to, the rest of the housing and that the volute casing is stationary with the rest of the static element.

It is understood that each of the impeller and the additional impeller may be centred on the housing axis. Any of the impellers may be a closed impeller, or more specifically have a rear hub plate and forward hub plate that supports a plurality of vanes between them and with the forward plate forming an impeller eye centred on the housing axis and arranged to receive the fluid.

The centrifuge separator, or more generally the system, may be arranged to form a lighter phase, or low-density fraction, of the fluid and a heavier phase, or a high-density fraction, of the fluid within the housing, or in the inner space, at a rotation of the housing. It is understood that the rotation is around the housing axis. The centrifuge separator, or more generally the system, may be arranged to discharge the lighter phase of the fluid through the outlet. It may further be arranged to discharge the heavier phase of the fluid through the additional outlet, or through the abovementioned ejection ports.

The centrifuge separator, or more generally the system, may further comprise a partition, or top disc, arranged to guide, or lead, the lighter phase of the fluid to, or towards, the outlet or to the pump, and the heavier phase to, or towards, the additional outlet or to the additional pump. It is understood that the partition at least in part is located within the housing or in the inner space. The partition may be arranged to separate the lighter phase of the fluid from the heavier phase of the fluid. The partition may be rotationally locked, of fixed to, the housing or to the upper bowl body.

The partition may be annular and centred on the housing axis. It may be located at the upper bowl body and arranged to allow the heavier phase to pass between the partition and the upper bowl body.

The centrifuge separator, or more generally the system, may comprise a plurality of discs, or bowl discs, arranged to, or configured to, enhance the formation of the lighter phase and the heavier phase of the fluid. It is understood that the discs are located within the housing or in the inner space. The discs may be annular, centred on the housing axis, and arranged in a disc stack aligned with the housing axis. Each disc may outline a truncated cone, or be frusto-conical, with a narrow end facing upward and a wide end facing downward. Each disc may have a central aperture at the housing axis, or at its narrow end. The core of the distributer, which is further specified below, may extend through the aperture.

Each pair of neighbouring discs may form an intermediate space between them. It is understood that the fluid is present in the intermediate space in operation.

The centrifuge separator, or more generally the system, may comprise a plurality of spacers, or caulks, wherein each pair of neighbouring discs may be interconnected by and spaced apart by one or more of the spacers. Each spacer interconnecting a pair of discs may be formed by, or be attached to, one of the discs of the pair. Each spacer interconnecting a pair of discs may electrically connect the discs of the pair. Worded differently, each spacer may be electrically conductive. It may be of an electrically conductive material. For example, the spacer may be of metal, such as steel. The spacer may be elongated and oriented radially relative to the housing axis. This contributes to a more even distribution of an electric current in the disc stack. For example, each spacer may be a strip of metal welded onto one of the discs. Alternatively, it may be formed by a disc as such, for example by a disc forming a bulge protruding towards the neighbouring disc.

Each pair of neighbouring intermediate spaces may be connected by a disc aperture in, or formed by, the disc positioned between them, or the disc contributing to form both intermediate spaces of the pair. It is understood that the fluid can flow through the aperture in operation.

The centrifuge separator, or more generally the system, may comprise a distributer, or disc carrier, arranged to, or configured to, distribute a flow of the fluid received via the inlet within the housing or in the inner space. It is understood that the distributer at least in part is located within the housing or in the inner space. The distributer may have a rotational symmetry relative to the housing axis. The distributer may be arranged to, or configured to, lead a flow of the fluid to the plurality of discs, or to the disc stack. The distributer may have, of form, a distributer cone arranged to, or configured to, face a flow of the fluid from the inlet. The distributer may be rotationally locked, of fixed to, the housing or to the lower bowl body.

The distributer may support the abovementioned discs or disc stack. More specifically, the distributor may have, or form, a core extending though each disc and aligning the disc stack with the housing axis. It is understood that the core is centred on the housing axis and that the core contacts the discs. The core may form a plurality of radial fins, or radial ribs, that extend along the housing axis, wherein each radial fin contacts the discs. Each pair of radial fins may form a gap between them that allows passage of the fluid from the disc stack, or from the intermediate space between each pair of neighbouring discs, toward to the outlet.

The inner space may be divided into chambers. It is further understood that the chambers may be in fluid communication with one another. The distributer may form, or delimit, an inlet chamber at the housing axis that is in fluid communication with the inlet. The distributer and the housing may jointly form, or delimit, a separation chamber that is in fluid communication with the inlet chamber. If the partition is present, the distributer, and partition may jointly form, or delimit, a separation chamber. The separation chamber may surround the inlet chamber, or it may be located radially outside the inlet chamber. It is understood that the abovementioned discs are located in the separation chamber. The distributer may form radially extending channels by which the inlet chamber is in fluid communication with the separation chamber. If the centrifuge separator, or more generally the system, has no partition, the housing and the distributer may jointly form, or delimit, an outlet chamber that is in fluid communication with the separation chamber and the outlet. If the centrifuge separator, or more generally the system, has a partition, the partition and the distributer may jointly form, or delimit, an outlet chamber that is in fluid communication with the separation chamber and the outlet. The outlet chamber may be located above the inlet chamber, at the housing axis, and/or radially inside the separation chamber. The partition and the housing may jointly form, or delimit, an additional outlet chamber that is in fluid communication with the separation chamber and the additional outlet. The additional outlet chamber may be located above the inlet chamber, above the outlet chamber, and/or radially outside the outlet chamber.

It is specified above that the housing is electrically conductive. If present, the housing shaft forms part of the housing and is also electrically conductive. The housing shaft may also form part the electric circuit, which is advantageous if the housing shaft forms the inlet of the housing. It is also specified that the static element may be electrically conductive and may form part of the abovementioned electric circuit. The abovementioned inlet pipe, paring disc, additional paring disc, outlet pipe, additional outlet pipe, and/or volute casing of the static element may thus be electrically conductive and form part the electric circuit. For example, each of the listed elements may be of metal, such as steel. The paring chamber, additional paring chamber, impeller, and additional impeller are formed by the housing and may thus be electrically conductive and form part the electric circuit.

The partition, the discs or disc stack, and/or the distributer specified above may be electrically conductive and form part of the electric circuit. For example, each of the elements may be of metal, such as steel. More specifically, the distributer cone, core, and radial fins of the distributer may be electrically conductive and form part of the electric circuit.

It is specified above that the static element and the housing may be electrically coupled in an electric network. The electric network may be arranged to distribute the current between the housing and the static element. The abovementioned inlet pipe, paring disc, additional paring disc, outlet pipe, additional outlet pipe, and/or volute casing of the static element may thus be coupled in the electric network. The paring chamber, additional paring chamber, impeller, and additional impeller are formed by the housing and may thus be coupled in the electric network.

The partition, discs or disc stack, and/or distributer specified above may be electrically coupled in the electric network. More specifically, the distributer cone, core, and radial fins of the distributer may be electrically coupled in the electric network.

The partition may contact, or be electrically coupled to, the housing, or more specifically to the upper bowl body. An upper part of the partition may contact the housing, and a lower part of the partition may contact, or be electrically coupled to, the housing and/or the disc stack. A radial inner part of the partition may contact, or be electrically coupled to, the housing and/or the disc stack and a radial outer part of the partition may contact, or be electrically coupled to, the housing and/or the disc stack.

The distributer may contact, or be electrically coupled to, the housing, the disc stack, and/or the partition. An upper part of the distributer, such as an upper part of the core or the radial fins, may contact, or be electrically coupled to, the partition and/or the housing, or more specifically the upper bowl body. A middle part of the distributer, such as the core or the radial fins, may contact, or be electrically coupled to, the disc stack. A lower part of the distributer may contact, or be electrically coupled to, the disc stack and/or the housing, or more specifically the lower bowl body and/or the housing shaft. It is understood that the middle part is located between the upper part and the lower part. A radially outer part of the distributer may contact, or be electrically coupled to, the disc stack. A radially inner part of the distributer may contact, or be electrically coupled to, the housing, or more specifically the lower bowl body and/or the housing shaft.

An upper part of the disc stack may contact, or be electrically coupled to, the housing and/or the partition. A radially outer part of the disc stack may contact, or be electrically coupled to, the housing and/or the partition. A lower part of the disc stack may contact, or be electrically coupled to, the distributer. A radially inner part of the disc stack may contact, or be electrically coupled to, the distributer, or more specifically to the core or the radial fins of the distributer. A radially outer part of the disc stack may contact, or be electrically coupled to, the distributer.

All relative positions presented above are understood to be with respect to a vertical housing axis. For each of the electrically coupled elements described above, such as the electrically coupled partition and housing, the centrifuge separator, or more generally the system, may comprise a coupling element that connects the elements or couples the elements together. It is understood that the coupling element is electrically conductive. For example, it may be of metal, such as steel.

It is understood that the abovementioned housing, partition, discs, disc stack, and distributer may be rotationally locked. This means that the components cannot rotate relative to one another, for example around the housing axis. The housing shaft, upper bowl body, lower bowl body, sliding piston of the housing may also be rotationally locked to one another.

In a second aspect of the proposed technology, which is not a part of the claimed invention, a method of inhibiting, reducing, or preventing microbiological growth in the system, or more specifically in the housing of the system, according to the first aspect is proposed. The method comprises: operating the power source to supply the current.

### Brief description of the drawings

A more complete understanding of the abovementioned and other features and advantages of the technology proposed herein will be apparent from the following detailed description of preferred embodiments in conjunction with the appended drawings, wherein:
Fig. 1 is a schematic view of a system with a centrifuge separator,
Fig. 2 is a schematic view of a system with another centrifuge separator,
Fig. 3 is a schematic view of a system with another centrifuge separator, and
Fig. 4 is a schematic view of a system with another centrifuge separator.

### Detailed description

In the figures and the below description, the same reference numerals are used for the same or related features.

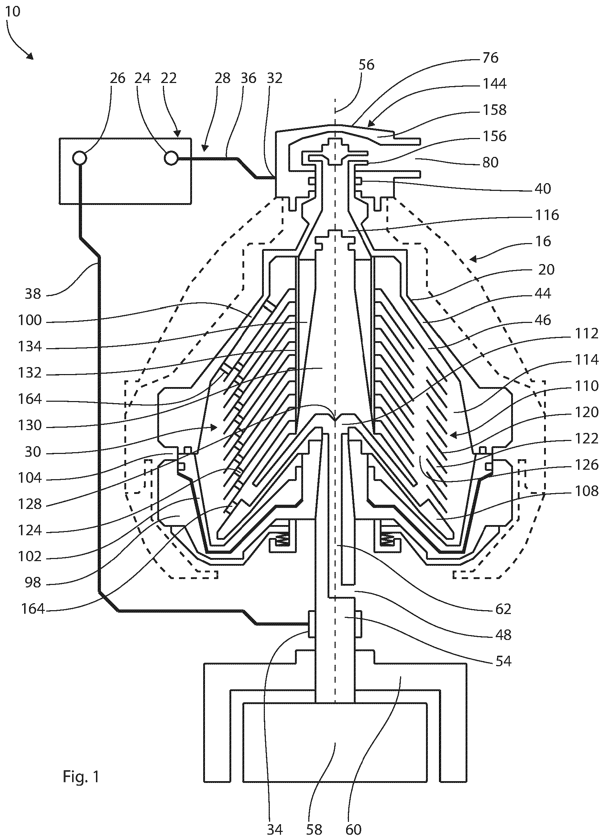

**Fig. 1** shows an embodiment of a system including a centrifuge separator 16 and an electric power source 22. The separator is shown in cross section. The separator 16 is shown in cross section and has a housing 20 of steel that is electrically conductive and configured to hold a liquid. The housing 20 has an electrically conductive wall 44 exposed to the fluid that forms an inner space 46 holding the fluid. The housing 20 is a rotating housing that can rotate around a vertical housing axis 56.

The housing 20 is rotationally symmetric relative to the housing axis 56. It has an annular lower bowl body 98 and an annular upper bowl body 100 centred on the housing axis 56. The lower bowl body 98 and the upper bowl body are electrically connected 100. The housing 20 has a housing shaft 54 in the form of a spindle that is fixed to the lower bowl body 98 and centred on the housing axis 56. The system 10 further has a support structure 60 that rotationally supports the housing 20 by the housing shaft 54. The housing 20 is electrically connected to the support structure 60 by a rotating element bearing (not shown). The system 10 further has a housing motor 58 coupled to the housing shaft 54 by which the housing 20 can be rotated. The housing shaft 54 forms an inlet 48 that can receive the liquid.

The housing further has a sliding piston 102 that can slide relative to the lower bowl body 98 parallel to the housing axis 56. The sliding piston 102 has an upper position in which it seals to the upper bowl 100 body and a lower position in which a gap is formed between the sliding piston 102 and the upper bowl body 100 through which sludge accumulated from the liquid can be ejected from the inner space 46. The lower bowl body 98 forms a plurality of ejection ports 104 through which accumulated slutch can pass after being ejected from the inner space 46. The sliding piston 102 is electrically connected to the lower bowl body 98. In the upper position, the sliding piston 102 is also electrically connected to the upper bowl body 100.

The centrifuge separator 16 forms a lighter phase of the fluid and a heavier phase of the liquid in the inner space 46 of the housing 20 at a rotation of the housing 20, with the lighter phase being closer to the housing axis 56 than the heavier phase. The centrifuge 16 has discs 120 located in the inner space 46 that enhance the formation of the lighter phase and the heavier phase. The discs 120 are annular and centred on the housing axis 56. They are arranged in a disc stack 110 that is aligned with the housing axis 56. Each disc 120 outlines a truncated cone with the narrow end facing upward and the wide end facing downward. Each disc 120 has a central aperture at it narrow end centred on the housing axis 56.

Each pair of neighbouring discs 120 forms an intermediate space 122 between them. The discs of the pair are spaced apart by a spacer 124 in the form of a small bulge formed by one of the discs 120. In an alternative embodiment, the spacer 124 is a radially oriented metal strip welded onto one of the discs. The spacer 124 electrically connects the discs 120 of the pair. Each pair of neighbouring intermediate spacers 124 are connected by a disc aperture 126 formed by the disc 120 positioned between them.

The centrifuge separator 16 further has a distributer 108 located in the inner space 46. The distributer 108 has a rotational symmetry relative to the housing axis 56. It is connected to the inlet 48 via a fluid conduit 62 formed by the housing shaft 54 and distributes the fluid to the disc stack 110. The distributer has distributer cone 128 that faces the flow of the fluid from the inlet 48. The distributer 108 is further a disc carrier and has a core 130 centred on the housing axis that extends through the central aperture of each disc 120 and aligns the disc stack 110 with the housing axis 56. The core 130 forms radial fins 132 that extend along the housing axis 56 and each radial fin 132 contacts the discs 120. Each pair of radial fins 132 form a gap 134 between them that allows passage of the fluid from the intermediate spaces 122 between the discs 120.

The system 10 further has a static element 76 that is stationary in operation of the centrifuge separator 16. The static element 76 is of steel and is electrically conductive. The static element 76 forms an outlet 80 for dispelling the fluid from the housing 20. The static element 76 is located outside the housing 20, the housing 20 extends into the static element 76, and the outlet 80 is located outside the inner space 46 formed by the housing 20. The housing 20 forms a closed impeller 156 and the static element 76 forms a volute casing 158 that cooperates to form a centrifugal pump 144 that pumps the fluid through the outlet 80 at a rotation of the housing 20.

The inner space 46 is divided into several chambers. The distributer 108 forms an inlet chamber 112 at the housing axis 56 that is in fluid communication with the inlet 48. The distributer 108 and the housing 20 jointly form a separation chamber 114 that is in fluid communication with the inlet chamber 112 by channels formed by the distributer 108.

The separation chamber 114 surrounds the inlet chamber 112 and the discs 120 are located in the separation chamber 114. The housing 20 and the distributer 108 jointly form an outlet chamber 116 located above the inlet chamber 112 and radially inside the separation chamber 114. The outlet chamber 116 is in fluid communication with the separation chamber via the gaps 134 formed by the radial fins 132. The outlet chamber 116 is further in fluid communication with the outlet 80 via the pump 144.

The discs 120, disc stack 110, and distributer 108 are of steel and are electrically conductive. An upper part of the radial fins 132 of the core 130 of the distributer 108 contacts and is electrically coupled to the upper bowl body 100 of the housing 20. At a middle part of the distributer 108, the radial fins 132 contact and are electrically coupled to the disc stack 110. A lower part and radially inner part of the distributer 108 contacts and is electrically coupled to the lower bowl body 98 and the housing shaft 54 of the housing 20. This contributes to distribute a current from the power source 22 to the disc stack and the distributer.

A lower and a radially outer part of the distributer 108 is electrically coupled to a lower and radially outer part of the disc stack 110 by a coupling element 164 of steel. An upper and radially outer part of the disc stack 110 is electrically coupled to the upper bowl body 100 by another coupling element 164 of steel. A radially outer part of the disc stack 110 is electrically coupled to the upper bowl body 100 by another a coupling element 164 of steel. This contributes to distribute a current from the power source 22 to the disc stack.

The system 10 further has a first internal connector 40 in the form of an electrically conductive seal of metal that connects the static element 76 and the upper bowl body 100 of the housing 20 and inhibits leakage between the upper bowl body 100 and the static element 76. The static element 76, upper bowl body 100, discs 120, disc stack 110, distributer 108, lower bowl body 98, sliding piston 102, and housing shaft 54 are thus electrically coupled in and form part of the electric network 30.

The system 10 has a first connector 32 in the form of a ring lug connector secured to the static element 76 by a screw. The system 10 further has a second connector 32 in the form of a slip ring connecting to the housing shaft 54. The system 10 further has an electric first wire 36 connecting the first connector 32 to a first terminal 24 of the power source 22 and an electric second wire 38 connecting the second connector 34 to a second terminal 26 of the power source 22. In an alternative embodiment, the second terminal 26 is connected to ground and housing shaft 54 is connected to ground via the support structure 60.

The power source 22 can supply an alternating current with a square waveform. The current is about 0.5 mA, has a frequency below of 1 Hz, and is supplied at a peak voltage of 50 V. The supplied current contributes to reduce microbiological growth in the centrifuge separator 16.

With the power source 22, first wire 36, first connector 32, static element 76, housing 20, second connector 34, and second wire 38 arranged as described here, the static element 76, housing 20, and electric power source 22 are operationally coupled in an electric circuit 28. The abovementioned electric network 30 forms part of the electric circuit 28. For example, the discs 120, disc stack 110, and distributer 108 are form part of the electric circuit 28.

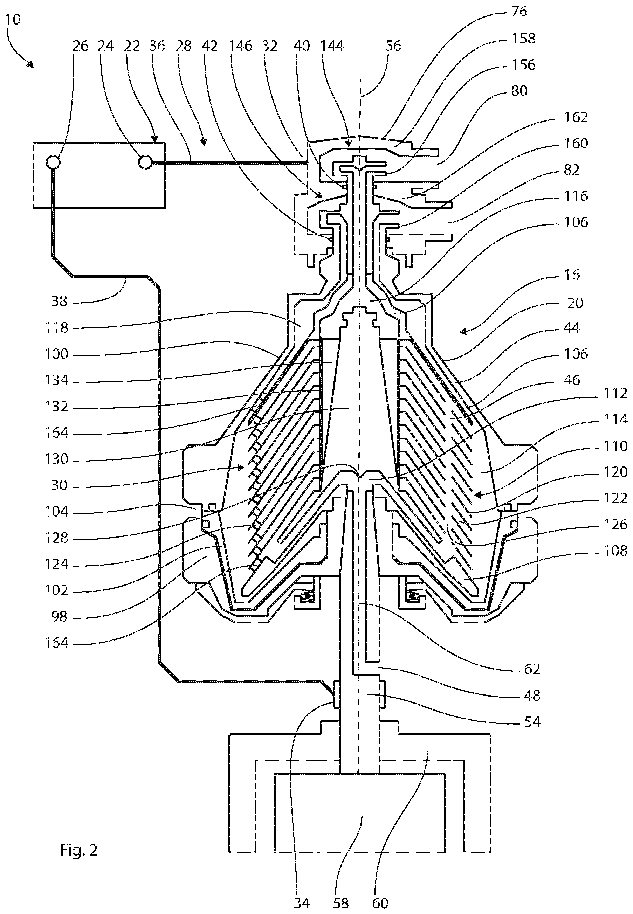

**Fig. 2** shows another embodiment of a system 10 including centrifuge separator 16, an electric power source 22, housing motor 58, and a support structure 60. The centrifuge separator 16 differs from the one in Fig. 1 by the static element 76 forming an additional outlet 80 and an additional volute casing 162 located below the volute casing 158. The housing 20 forms a closed additional impeller 160 that cooperates with the additional volute casing 162 to form an additional centrifugal pump 146 that can pump the fluid through the additional outlet 80 at a rotation of the housing 20.

The centrifuge separator 16 further has an annular partition 106 that is located within inner space 46 of the housing 20 and centred on the housing axis 56. The partition 106 guides the lighter phase of the liquid to the pump 144 and the heavier phase of the liquid to the additional pump 146. The partition 106 is located at the upper bowl body 100 and the heavier phase of the liquid passes between the partition 106 and the upper bowl body 100.

Instead of the housing 20 and the distributer 108 forming the outlet chamber 116, it is formed by the partition 106 and the distributer 108. As in the embodiment of Fig. 1, the outlet chamber 116 is in fluid communication with the separation chamber 114 and the outlet 80 via the pump 144. The partition 106 and the upper bowl body 100 of the housing 20 jointly form an additional outlet chamber 118 that is in fluid communication with the separation chamber 114 and the additional outlet 80 via the additional pump 146. The additional outlet chamber 118 is located above and radially outside the outlet chamber 116.

The partition 106 is of steel and is electrically conductive. An upper and radially inner part of the partition 106 contacts and is electrically coupled to the upper bowl body 100 of the housing 20. A radial outer part of the partition 106 is electrically coupled to the housing 20 and the disc stack 110 by coupling elements 164 of steel. Instead of contacting the housing 20, an upper part of the radial fins 132 of the core 130 of the distributer 108 contacts and is electrically coupled to the partition 106.

The system 10 further has a first internal connector 40 in the form of electrically conductive seals of metal. Both seals connect the static element 76 and the housing 20 and inhibits leakage between the static element 76 and the housing 20. The upper seal inhibits fluid to pass between the volute casing 158 and the additional volute casing 162. The lower seal inhibits fluid to pass to the outside of the housing 20. The static element 76, upper bowl body 100, partition 106, discs 120, disc stack 110, distributer 108, lower bowl body 98, sliding piston 102, and housing shaft 54 are thus electrically coupled in and form part of the electric network 30.

The power source 22, the first wire 36, the first connector 32, the second connector 34, and the second wire 38 are arranged as described in relation to Fig. 1. In an alternative embodiment, the second terminal 26 of the power source 22 is connected to ground and housing shaft 54 is connected to ground via the support structure 60.

With the components of the system 10 arranged as described above, the static element 76, housing 20, and electric power source 22 are operationally coupled in an electric circuit 28. The abovementioned electric network 30 forms part of the electric circuit 28. For example, the partition 106, discs 120, disc stack 110, and distributer 108 are parts of the electric circuit 28.

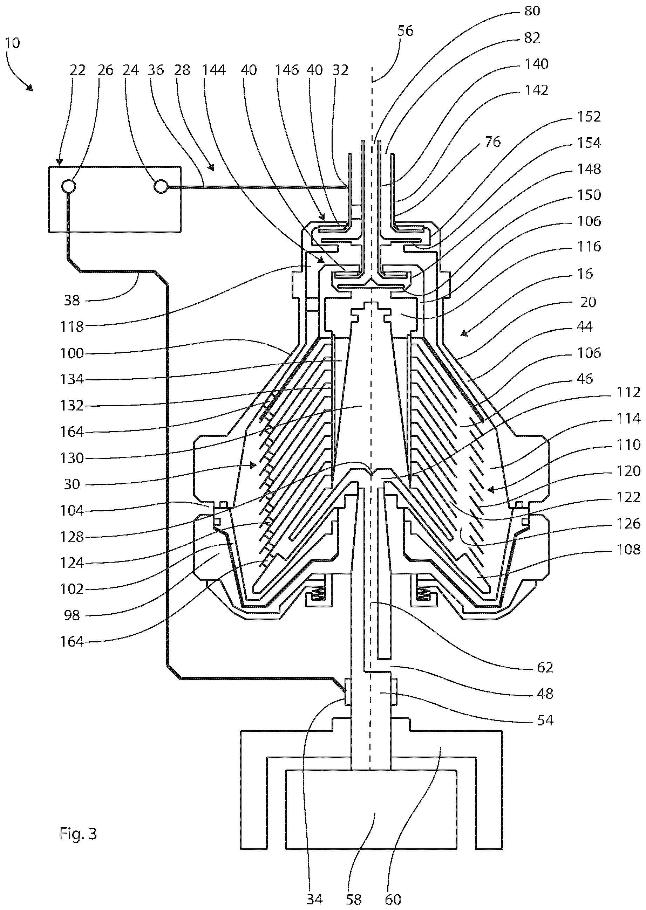

**Fig. 3** shows another embodiment of a system 10 including centrifuge separator 16, an electric power source 22, housing motor 58, and a support structure 60. The centrifuge separator 16 differs from the one in Fig. 2 by the static element 76 extending into the housing 20 and being located partially in the inner space 46 of the housing 20. The outlet 80 and the additional outlet 80 are located outside the housing 20, as in the embodiment of Fig. 2.

The upper bowl body 100 of the housing 20 forms a paring chamber 148 and the static element forms a paring disc 150 that cooperate to form a centripetal pump 144 that can pump the liquid through the outlet 80 at a rotation of the housing 20. The static element 76 forms an outlet pipe 140 with an inner open end coupled to the paring disc 150 of the centripetal pump 144 and an outer open end constituting the outlet 80. The outlet pipe 140 is centred on the housing axis 56. The housing 20 further forms an additional paring chamber 152 and the static element further forms an additional paring disc 154 that cooperate to form an additional centripetal pump 146 that can pump the liquid through the additional outlet 82 at a rotation of the housing 20. The static element 76 forms an additional outlet pipe 142 that is concentric with, surrounds, and is electrically connected to the outlet pipe 140. It has an inner open end coupled to the additional paring disc 154 of the additional centripetal pump 146 and an outer open end constituting the additional outlet 82.

The first internal connector 40 includes electrically conductive seals of metal. Both seals connect the static element 76 and the housing 20 and inhibit gas from entering the housing between the static element 76 and the housing 20. The upper seal inhibits gas from entering the housing 20 between the additional paring disc 154 and the additional paring chamber 152, and the lower seal inhibits gas from entering the housing 20 between the paring disc 150 and the paring chamber 148. The static element 76, upper bowl body 100, partition 106, discs 120, disc stack 110, distributer 108, lower bowl body 98, sliding piston 102, and housing shaft 54 are thus electrically coupled in and form part of the electric network 30. The inlet pipe 138, paring disc 150, additional paring disc 154, outlet pipe 140, and additional outlet pipe 142, of the static element 76 are of steel and electrically conductive and form part the electric network 30. The paring chamber 148 and the additional paring chamber 152 of the housing 20 also form part the electric network 30.

The power source 22, the first wire 36, the first connector 32, the second connector 34, and the second wire 38 are arranged as described in relation to Fig. 1, with the only difference that the first connector 32 connects specifically to the additional outlet pipe 142. In an alternative embodiment, the second terminal 26 of the power source 22 is connected to ground and the housing shaft 54 is connected to ground via the support structure 60. Arranged this way, the static element 76, housing 20, and electric power source 22 are operationally coupled in an electric circuit 28, and the parts of the electric network 30 also form part of the electric circuit 28.

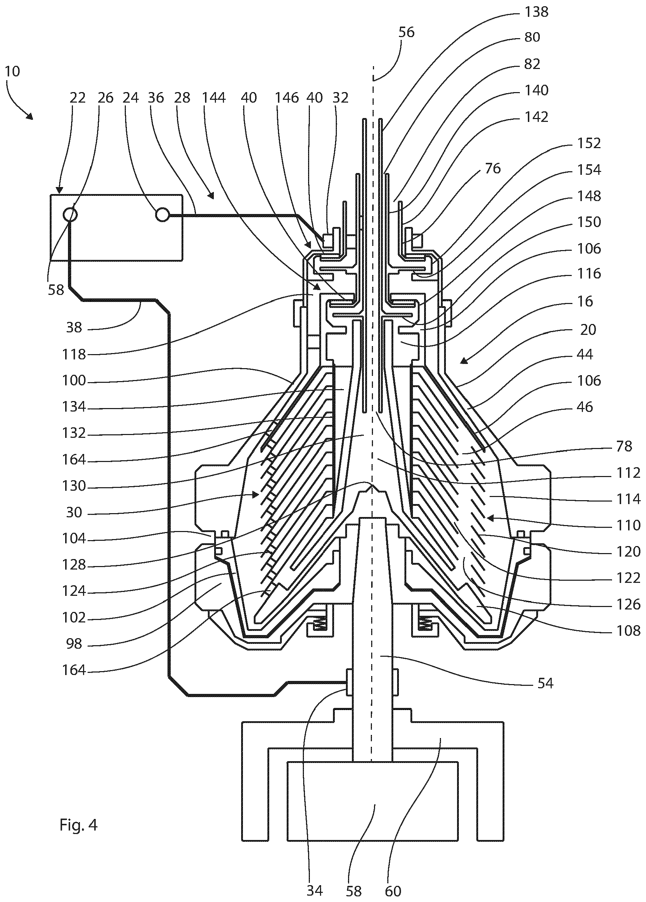

**Fig. 4** shows another embodiment of a system 10 including centrifuge separator 16, an electric power source 22, housing motor 58, and a support structure 60. The centrifuge separator 16 differs from the one in Fig. 3 by the static element 76 further forming an inlet pipe 138 with an inlet 78 for introducing the liquid into the housing 20, instead of the housing shaft 54 forming the inlet 78. The inlet pipe 138 is centred on the housing axis 56 and extends from outside the housing 20 into the inlet chamber 112 formed by the distributer 108. The inlet pipe 138 has an outer open end located outside the housing 20 that receives the fluid and an inner open end that forms an inlet 78 inside the housing 20. The outlet pipe 140 and the additional outlet pipe 142 are concentric with and surround the inlet pipe 138. The outlet pipe 140 is connected and electrically coupled to the inlet pipe 138, which forms part of the electric network 30.

The system 10 further differs by the first connector 32 being a slip ring that connects to the upper bowl body 100 of the housing 20.

The power source 22, the first wire 36, the first connector 32, the second connector 34, and the second wire 38 are arranged as described in relation to Fig. 1, with the inner space 46 of the housing 20 located between the first connector 32 and the second connector 34. In an alternative embodiment, the second terminal 26 of the power source 22 is connected to ground and the housing shaft 54 is connected to ground via the support structure 60. The static element 76, housing 20, and electric power source 22 are thus operationally coupled in an electric circuit 28, and the parts of the electric network 30 also form part of the electric circuit 28.

### Item list

10 system
16 separator
20 housing
22 electric power source
24 first terminal
26 second terminal
28 electric circuit
30 electric network
32 first connector
34 second connector
36 electric first wire
38 electric second wire
40 first internal connector
42 second internal connector
44 wall
46 inner space
48 inlet
50 outlet
54 housing shaft
56 housing axis
58 housing motor
60 support structure
62 fluid conduit
76 static element
78 inlet
80 outlet
82 additional outlet
98 lower bowl body
100 upper bowl body
102 sliding piston
104 ejection ports
106 partition
108 distributer
110 disc stack
112 inlet chamber
114 separation chamber
116 outlet chamber
118 additional outlet chamber
120 disc
122 intermediate space
124 spacer
126 disc aperture
128 distributer cone
130 core
132 radial fins
134 gap
138 inlet pipe
140 outlet pipe
142 additional outlet pipe
144 pump
146 additional pump
148 paring chamber
150 paring disc
152 additional paring chamber
154 additional paring disc
156 impeller
158 volute casing
160 additional impeller
162 additional volute casing
164 coupling element

## Claims

1. A system (10) comprising:
- a housing (20),
- an electric power source (22), wherein
the housing (20) is electrically conductive and configured to hold a fluid, the power source (22) is arranged to supply an electric current, the housing (20) and the electric power source (22) are operationally coupled in an electric circuit (28) arranged to lead the current from the electric power source (22) through the housing (20), and the electric current is configured to inhibit microbiological growth within the housing (20),
**characterized in that** the system (10) further comprises:
- a static element (76), wherein
the housing (20) is a rotating housing (20) arranged to rotate around a housing axis (56), the static element (76) and the housing (20) are electrically coupled in an electric network (30) that forms part of the electric circuit (28).

2. The system (10) according to claim 1, wherein the system (10) comprises:
- a first internal connector (40) that electrically couples the static element (76) to the housing (20), and the first internal connector (40) is a rotary electrical connector.

3. The system (10) according to claim 1 or 2, wherein the housing (20) and the static element (76) form part of a centrifuge separator (16), and the centrifuge separator (16) is arranged to form a lighter phase of the fluid and a heavier phase of the fluid within the housing (20) at a rotation of the housing (20).

4. The system (10) according to claim 3 , wherein the housing (20) comprises a housing shaft (54), the housing shaft (54) is centred on the housing axis (56), the housing (20) has an inlet (48) for receiving the fluid into the housing (20), and the inlet (48) is formed by the housing shaft (54).

5. The system (10) according to claim 3 or 4, wherein the static element (76) forms an outlet (80) for dispelling the fluid from the housing (20).

6. The system (10) according to any of the claims 3 to 5, wherein the centrifuge separator (16) comprises:
- a plurality of discs (120), wherein
the discs (120) are configured to enhance the formation of the lighter phase and the heavier phase of the fluid, the discs (120) are annular, centred on the housing axis (56), and arranged in a disc stack (110) aligned with the housing axis (56), and the discs (120) are electrically coupled in the electric network (30).

7. The system (10) according to any of the claims 1 to 6, wherein the electric circuit (28) is connected to ground.

8. The system (10) according to any of the claims 1 to 7, wherein the current is an alternating current, the current has a square waveform, the current is below 10 mA, and the current is greater than 0.01 mA.

## Patentansprüche

1. System (10), umfassend:
- ein Gehäuse (20),
- eine elektrische Energiequelle (22), wobei
das Gehäuse (20) elektrisch leitend und so konfiguriert ist, dass es ein Fluid enthält, die Stromquelle (22) so angeordnet ist, dass sie einen elektrischen Strom liefert, das Gehäuse (20) und die elektrische Stromquelle (22) betriebsmäßig in einem Stromkreis (28) gekoppelt sind, der so angeordnet ist, dass er den Strom von der elektrischen Stromquelle (22) durch das Gehäuse (20) leitet, und der elektrische Strom so konfiguriert ist, dass er mikrobiologisches Wachstum innerhalb des Gehäuses (20) hemmt,
**dadurch gekennzeichnet, dass** das System (10) ferner umfasst:
- ein statisches Element (76), wobei
das Gehäuse (20) ein rotierendes Gehäuse (20) ist, das so angeordnet ist, dass es sich um eine Gehäuseachse (56) dreht, das statische Element (76) und das Gehäuse (20) elektrisch in einem elektrischen Netzwerk (30) gekoppelt sind, das einen Teil des Stromkreises (28) bildet.

2. System (10) nach Anspruch 1, wobei das System (10) umfasst:
- einen ersten internen Verbinder (40), der das statische Element (76) mit dem Gehäuse (20) elektrisch koppelt, und der erste interne Verbinder (40) ein elektrischer Drehverbinder ist.

3. System (10) nach Anspruch 1 oder 2, wobei das Gehäuse (20) und das statische Element (76) Teil eines Zentrifugenabscheiders (16) sind und der Zentrifugenabscheider (16) so angeordnet ist, dass er bei einer Drehung des Gehäuses (20) eine leichtere Phase des Fluids und eine schwerere Phase des Fluids innerhalb des Gehäuses (20) bildet.

4. System (10) nach Anspruch 3, wobei das Gehäuse (20) eine Gehäusewelle (54) umfasst, die Gehäusewelle (54) auf der Gehäuseachse (56) zentriert ist, das Gehäuse (20) einen Einlass (48) zum Aufnehmen des Fluids in das Gehäuse (20) aufweist, und der Einlass (48) von der Gehäusewelle (54) gebildet wird.

5. System (10) nach Anspruch 3 oder 4, wobei das statische Element (76) einen Auslass (80) zum Austragen des Fluids aus dem Gehäuse (20) bildet.

6. System (10) nach einem der Ansprüche 3 bis 5, wobei der Zentrifugenseparator (16) umfasst:
- eine Vielzahl von Scheiben (120), wobei
die Scheiben (120) so konfiguriert sind, dass sie die Bildung der leichteren Phase und der schwereren Phase des Fluids verbessern, die Scheiben (120) ringförmig sind, auf der Gehäuseachse (56) zentriert sind und in einem Scheibenstapel (110) angeordnet sind, der mit der Gehäuseachse (56) ausgerichtet ist, und die Scheiben (120) in dem elektrischen Netzwerk (30) elektrisch gekoppelt sind.

7. System (10) nach einem der Ansprüche 1 bis 6, wobei der Stromkreis (28) mit der Masse verbunden ist.

8. System (10) nach einem der Ansprüche 1 bis 7, wobei der Strom ein Wechselstrom ist, der Strom eine Rechteckwellenform hat, der Strom unter 10 mA liegt und der Strom größer als 0,01 mA ist.

## Revendications

1. Système (10) comprenant :
- un boîtier (20),
- une source de puissance électrique (22), dans lequel
le boîtier (20) est électriquement conducteur et configuré pour contenir un fluide, la source de puissance (22) est agencée pour alimenter un courant électrique, le boîtier (20) et la source de puissance électrique (22) sont couplés de manière opérationnelle dans un circuit électrique (28) agencé pour conduire le courant de la source de puissance électrique (22) à travers le boîtier (20), et le courant électrique est configuré pour inhiber la croissance microbiologique à l'intérieur du boîtier (20),
**caractérisé en ce que** le système (10) comprend également :
- un élément statique (76), dans lequel
le boîtier (20) est un boîtier rotatif (20) agencé pour tourner autour d'un axe de boîtier (56), l'élément statique (76) et le boîtier (20) sont couplés électriquement dans un réseau électrique (30) qui fait partie du circuit électrique (28).

2. Système (10) selon la revendication 1, dans lequel le système (10) comprend :
- un premier connecteur interne (40) qui couple électriquement l'élément statique (76) au boîtier (20), et le premier connecteur interne (40) est un connecteur électrique rotatif.

3. Système (10) selon la revendication 1 ou 2, dans lequel le boîtier (20) et l'élément statique (76) font partie d'un séparateur centrifuge (16), et le séparateur centrifuge (16) est agencé pour former une phase plus légère du fluide et une phase plus lourde du fluide à l'intérieur du boîtier (20) lors d'une rotation du boîtier (20).

4. Système (10) selon la revendication 3, dans lequel le boîtier (20) comprend un arbre de boîtier (54), l'arbre de boîtier (54) est centré sur l'axe de boîtier (56), le boîtier (20) a une entrée (48) pour recevoir le fluide dans le boîtier (20), et l'entrée (48) est formée par l'arbre de boîtier (54).

5. Système (10) selon la revendication 3 ou 4, dans lequel l'élément statique (76) forme une sortie (80) pour évacuer le fluide du boîtier (20).

6. Système (10) selon l'une quelconque des revendications 3 à 5, dans lequel le séparateur centrifuge (16) comprend :
- une pluralité de disques (120), dans lequel
les disques (120) sont configurés pour favoriser la formation de la phase plus légère et de la phase plus lourde du fluide, les disques (120) sont annulaires, centrés sur l'axe du boîtier (56), et disposés dans une pile de disques (110) alignée avec l'axe du boîtier (56), et les disques (120) sont couplés électriquement dans le réseau électrique (30).

7. Système (10) selon l'une quelconque des revendications 1 à 6, dans lequel le circuit électrique (28) est connecté à la terre.

8. Système (10) selon l'une quelconque des revendications 1 à 7, dans lequel le courant est un courant alternatif, le courant a une forme d'onde carrée, le courant est inférieur à 10 mA et le courant est supérieur à 0,01 mA.
